# EUROPEAN PATENT APPLICATION

(11) **EP 2 550 974 A2**
(43) Date of publication of application: **30.01.2013**
(21) Application number: 11192023.7
(22) Date of filing: 17.05.2010
(51) Int. Cl.: A61K 39/145, C12N 15/863

(54) **Universal influenza vaccine based on recombinant modified vaccinia Ankara virus (MVA)**

(30) Priority: 18.05.2009 IN DE10152009
(62) Divisional of application: 10777464.8
(71) Applicant: Panacea Biotec Ltd., New Delhi 110 044 (IN)
(72) Inventor: Jain, Rajesh, 110 044 New Delhi (IN); Vinayak, Virender Kumar, 110 044 New Delhi (IN); Shukla, Nidhi, 110 044 New Delhi (IN); Aggarwal, Neeraj, 110 044 New Delhi (IN); Mehta, Rajan, 110 044 New Delhi (IN)
(74) Representative: Lecca, Patricia S.

(57) **Abstract**

The present invention relates to a novel influenza vaccine, a novel plasmid for preparing the same and a novel dosage form comprising the same. The present invention in particular relates to a recombinant modified vaccinia Ankara (MVA) virus comprising and capable of simultaneously expressing a cassette of at least four foreign genes from influenza virus, specifically an avian influenza virus, wherein the said genes are inserted at a non-essential site, within the MVA genome. The invention further relates to a recombinant modified vaccinia Ankara (MVA) virus comprising and capable of simultaneously expressing a cassette of not less than two foreign genes from influenza virus, wherein the said genes are inserted at a non-essential site, within the MVA genome, with the provision that at least one foreign gene is either PB2 or M2e. The invention also provides composition and methods of making the universal influenza vaccine.

## Description

### FIELD OF INVENTION

The present invention relates to a novel universal influenza vaccine and a novel dosage form comprising the same. The invention in particular relates to a recombinant modified vaccinia Ankara (MVA) virus comprising and capable of expressing a novel combination of foreign genes of influenza virus, specifically H5N1 avian influenza virus, preferably in a single cassette.

### BACKGROUND OF THE INVENTION

Influenza viruses belong to the family Orthomyxoviridae and are divided into three genera A, B and C. Influenza A viruses can infect birds as well as mammals whereas influenza B and C viruses can infect only human beings. These are enveloped viruses with segmented genome made of eight single-stranded negative RNA segments. Aquatic birds are natural reservoirs of influenza A viruses (Webster *et al.,* 1992). These viruses are known to cross the species barrier and cause either transitory infections or establish permanent lineages in mammals including man (Ludwig *et al.,* 1995). The most devastating flu viruses of the 20^{th} century, Spanish flu pandemic in 1918 (H1N1), Asian flu pandemic in 1957 (H2N2) and Hong Kong flu pandemic in 1968 (H3N2), were all of avian origin (Lipatov *et al.,* 2004).

Highly pathogenic avian influenza viruses of subtype H5N1, causing what is commonly called as "bird flu," are highly contagious and deadly pathogen in poultry. Since late 2003, H5N1 has reached epizootic levels in domestic fowls and has swept across almost half the world. However, its spread to the human population has so far been limited. The first case of H5N1 infection in humans was reported in 1997 in Hong Kong (Claas *et al.,* 1998; Subbarao et.al., 1998). In almost all the cases reported so far, evidence indicates bird-to-human transmission although there is a single report from Thailand that indicates direct human-to-human transmission (Ungchusak *et al.,* 2005).

Influenza pandemics are caused by "antigenic shift", which means major changes in the HA/NA or both genes leading to the formation of a new influenza A subtype not currently circulating in humans. The changes in hemagglutinin (HA), the principal viral protein that is responsible for binding to host cell receptors, is one of the key proteins where changes must occur for H5N1 virus to establish infection in humans (Gambaryan *et al.,* 2006; Stevens *et al* .2006; Yamada *et al.,* 2006). Subsequently, mutations must happen in the proteins of the viral polymerase complex for virus to replicate efficiently so that infection can perpetuate (Almond, 1977; Subbarao *et al.,* 1993; Hatta *et al.,* 2001; Maines *et al.,* 2005; Li Z *et al,* 2005; Subbarao *et al.,* 1998; Katz *et al.,* 2000). If changes in receptor specificity of H5N1 viruses coupled with replication potential in humans or reassortment with an already circulating human influenza virus do happen, then establishment of a permanent human lineage cannot be ruled out. Given the looming danger of a new influenza pandemic, it makes sense to use resources for pre-pandemic preparedness. This includes the efforts to develop an effective vaccine. Current vaccines for flu are either used by parenteral route or by intranasal route.

### Parenteral influenza vaccines

Since the introduction in 1940s of an inactivated influenza vaccine containing inactivated virus material from infected embryonated eggs, the risk and course of the infection as well as the mortality rates in elderly persons have dropped. There are three classes of inactivated vaccines: whole, split (chemically disrupted with ether or tributyl phosphate) and subunit (purified surface glycoproteins). These vaccines can be administrated intramuscularly or subcutaneously. Whole inactivated influenza vaccine is not currently used due to high levels of side effect. The seasonal influenza vaccine (split and subunit) is trivalent, comprising a strain each of H3N2, H1N1 influenza A virus and an influenza B virus. Over the last several years, at least one of the components had to be changed each year due to antigenic drift. The BEGRIVAC^{™}, FLUARIX^{™}, FLUZONE^{™} and FLUSHIELD^{™} products are split vaccines. The FLUVIRIN^{™}, AGRIPPAL^{™} and INFLUVAC^{™} products are subunit vaccines. Inactivated influenza vaccines are 60% to 100% effective in preventing morbidity and mortality, however, lower rates of efficacy are observed in the young and elderly.

In June 2003, the FDA approved the use of a cold-adapted live attenuated influenza virus vaccine, Flumist^{™}, developed by Medimmune Inc., in healthy children and adolescents, 5-17 years of age, and healthy adults, 18-49 years of age for seasonal influenza.

Recent studies have reported the use of reverse genetic engineering to produce vaccine strains bearing modified genes to attenuate virulence (Subbarao etal.2003).

### Intranasal Influenza vaccine:

M.L.Clements et al 1986, have previously reported that both secretory IgA and serum IgG participate in immunity to influenza virus. Moreover, in mice, a number of published studies have demonstrated the importance of respiratory IgA for protection against influenza infection. It has also been found that an advantage of stimulating a local IgA response to influenza is that it is often of a broader specificity than the serum response and thus can provide cross-protection against viruses possessing hemagglutinin molecules different from those present in the vaccine. Accordingly, influenza vaccines that elicit both local IgA and serum anti-hemagglutinin responses should provide superior immunity to current vaccines. However, parenteral vaccination (intramuscular, subcutaneous etc) is not effective at eliciting local IgA production, if there has been no previous mucosal exposure (e.g infection). In order to stimulate the mucosal immune system, the vaccine must be applied topically to a mucosal surface.

Mucosal administration of influenza vaccine has a number of advantages over traditional parenteral immunization regimes. Paramount amongst these is the more effective stimulation of the local mucosal immune system of the respiratory tract and the likelihood that vaccine uptake rates would be increased owing to the reduction in fear and discomfort associated with injections Accordingly, a number of attempts have been made to develop mucosal influenza vaccines. A drawback however is that inactivated vaccines are often poorly immunogenic when given mucosally. In order to overcome this problem, different approaches are being evaluated to improve the immunogenicity of flu vaccines given orally or intranasally. Some of these efforts include the use of the B subunit of cholera toxin (CTB) as an adjuvant, encapsulation of the vaccine in a variety of microspheres, and the use of live attenuated influenza strains.

The commercially available intranasal vaccine Flumist, for seasonal flu, was launched in USA in September 2003. MedImmune's FluMist is a live attenuated vaccine that is administered by nasal spray to patients between the ages of 5 and 49. This vaccine is not licensed for use in "at-risk" populations. The virus for this vaccine is also grown on embryonated chicken eggs and is a live attenuated formulation that is delivered by nasal spray. Besides limitations in amount of doses that can be manufactured each year, the vaccine is not licensed for use in the young and elderly populations, which need protection from influenza the most.

There are a number of other candidate vaccines for intra nasal administration that are currently under clinical evaluation. However, none of these prior art references even remotely suggest the combination of antigens and the process according to the current invention. Patent no. EP1214054B1 by GlaxoSmithKline Pharmaceuticals particularly relates to a non-live split, intranasal influenza vaccine. Sanofi Pasteur is collaborating with Avant Immunotherapeutics to use their Micromer delivery system for the development of an intranasal influenza vaccine. Micromer is a water based polymer utilizing polyphosphazene for the micro-encapsulation of vaccines and mucosal delivery. Currently this is in preclinical stage. Patent no.WO2003063785 by Symbigene Inc., claims a recombinant yeast based vaccine for the potential treatment of influenza. The vaccine may be administered orally or intranasally. Preclinical studies of the vaccine are ongoing in USA. Acambis has discontinued the development of an intranasal vaccine that was under evaluation for influenza prophylaxis. The vaccine incorporated inactivated influenza antigens and chitosan. Biovector Therapeutics' program to develop an intranasal mucosal vaccine against influenza, using its Biovector delivery technology, has been discontinued. BioSante Pharmaceuticals is developing a non-injectable, intranasal vaccine comprising an antigen from the H5N1 strain of influenza virus combined with BioSante's calcium phosphate nanoparticle based vaccine adjuvant, for the treatment of H5N1 influenza virus infection. NanoBio Corporation is developing NB006, a mucosal vaccine for influenza. Preclinical studies of NB006 are ongoing and an IND was filed in first quarter 2007. A phase I trial has begun in fourth quarter of 2007. Patent no. US7323183 by Archimedes Pharma (formerly West Pharmaceutical Services Inc.) relates to a vaccine targeted against influenza, using the company's proprietary ChiSys nasal delivery technology. Phase I evaluation which was being conducted in the UK (West, March 2004) has been completed. PCT application WO2009007244 by Green Hills Biotechnology (GHB) deals with FluVacc, a live attenuated replication deficient influenza virus vaccine. The vaccine lacks the pathogenecity factor NS1, is produced in Vero cells and is administered intranasally with a spray device. A phase I trial of FluVacc has been initiated in Austria. ID Biomedical Corp. is developing an intranasal vaccine, FluINsure, for the potential treatment of influenza (Patent no. US6743900). The vaccine is based on ID Biomedical's proprietary vaccine delivery/adjuvant technology, Proteosomes, in combination with a purified preparation of influenza proteins including hemagglutinin. A phase I study has commenced in Canada to study the safety and immunogenicity of its nasal proteosome influenza vaccine in humans. NasVax Ltd. is developing a vaccine for the prophylaxis of influenza which utilizes CCS, its proprietary polycationic lipid technology. CCS acts as an adjuvant and also allows intranasal administration of the vaccine. A phase I/II trial in 140 healthy volunteers has been completed in Israel and the company planned to file an application seeking permission to initiate a European phase III trial mid 2007. BioDiem Ltd., is developing an intranasally delivered single dose, cold adapted, live attenuated influenza virus vaccine for seasonal influenza virus infection. The vaccine is based on a vaccine that has been marketed in Russia and the CIS, since 1993 by the Institute of Experimental Medicine in St. Petersberg (Russia). Preclinical evaluation of the vaccine is ongoing in Europe and USA.

### Efforts to develop H5N1 vaccine:

Medimmune Inc. is developing a cold adapted H5N1 vaccine. MedImmune uses methods such as reverse genetics and classical reassortment to place hemagglutinin genes with pandemic potential into an attenuated human flu virus. MedImmune Inc. research team has created three candidate vaccines by combining modified proteins derived from virulent H5N1 flu viruses with proteins from an artificially weakened (attenuated) flu strain using reverse genetics. The virulent H5N1 viruses were isolated from human cases in Hong Kong in 1997 and 2003, and Vietnam in 2004 (A/Vietnam/1203/04). The attenuated flu vaccine strain, which also serves as the basis for MedImmune's FluMist® influenza vaccine, is lab-grown in progressively colder temperatures ("cold-adapted") to prevent the resulting vaccine viruses from spreading beyond the relatively cool upper respiratory tract. Large quantities of the resulting cold-adapted viruses were grown in chicken eggs. In June 2006, MedImmune launched a Phase 1 human volunteer study to evaluate the safety and immunogenicity of a live, attenuated H5N1 vaccine. Results from the study are not yet available. (www.medicalnewstoday.com/articles/51701.php)

A USFDA licensed H5N1 vaccine introduced by Sanofi Pasteur in April 2007 is an inactivated vaccine and is intended to be used in case of emergency only (www.fda.gov/bbs/topics/NEWS). The reassortant candidate vaccine strain NIBRG-14, used by Sanofi Pasteur was procured from National Institute for Biological Standard, UK who developed this strain using reverse genetics. Sinovac Biotech Ltd. (China) is currently developing an inactivated H5N1 vaccine using the reassortant candidate vaccine strain NIBRG-14 obtained from National Institute for Biological Standard, UK (www.medicalnewstoday.com/articles/51392.php). The virus is grown in eggs and after inactivation is given along with an adjuvant. This vaccine is in phase II clinical trials. (//www.bio-medicine.org/medicine-technology-1). Chiron Vaccines/Novartis Vaccines, Italy, is developing an egg based MF59 adjuvanted inactivated vaccine using a Vietnam 2004 strain. Chiron Corporation will produce the vaccine using identical production process as used for its marketed influenza vaccine Fluvirin. The phase II clinical trials of the vaccine were over in 2006. (www.ifpma.org/Influenza/content/pdfs/Table_Avian_Pandemic_Influenza_RnD_17 Oct06.pdf). DelSite Biotechnologies Inc. is developing an inactivated, nasal powder vaccine against H5N1 virus strain using its proprietary GelVac delivery system. The GelVac nasal powder vaccine delivery system is based on the company's GelSite polymer technology, a novel polysaccharide that turns from a powder to a gel upon contact with nasal fluids, resulting in controlled release and increased nasal residence time of vaccine antigens. DelSite Biotechnologies Inc has partnered with Invitrogen Corporation's PD-Direct services to develop a process to enable a cell based H5N1 whole virion antigen to be used for the nasal delivery. (www.biospace.com/news story.aspx?NewsEntityId=34335)

### Limitations of the current vaccines:

There are many limitations of the inactivated/split/cold adapted vaccines - the major limitation being that the candidate strain has to be changed every year depending upon the circulating strain at that time. Furthermore, the identified strain has to be adopted to grow to high titers in embryonated eggs. The use of later causes certain challenges such as the availability of year round supplies of high quality specific pathogen free eggs. The use of such eggs is a must because the presence of adventitious agents in eggs can jeopardize the preparation of influenza virus vaccines. In case of a flu epidemic in birds, supply of specific pathogen free eggs to cultivate the flu virus would become scarce. This would affect availability of the vaccine for use in human adversity. Additionally, hypersensitivity reactions due to egg proteins do occur in a significant population. Inactivation of virus by formalin also leads to denaturation of antigenic epitopes and thus likely to be less efficacious. Moreover, the efficacy of these vaccines is suboptimal because of limited ability to elicit local IgA and cytotoxic T cell responses. Hence the protective effect of the traditional split/subunit vaccines is very limited.

### Alternate influenza vaccines:

In an effort to alleviate the shortcomings of the currently manufactured influenza vaccines, several alternative approaches to produce vaccines are currently being developed.

### Cell culture based vaccines:

The use of cell culture based systems is probably the most investigated of the areas being pursued. The two main cell lines that are being tested are MDCK (Palache etal., 1999) and Vero (Halperin etal. 2002 and Nicolson, 2005). Baxter International Inc. initiated phase III clinical trials of its H5N1 (A/Vietnam/1203/04) inactivated whole cell vaccine in the year 2007. It is using its proprietary Vero cell technology for the cultivation of the viruses (www.medicalnewstoday.com/articles/66602.php). The procedure for inactivation of the virus grown in these cells for use in vaccines is the same as that used with egg produced virus. Therefore, the virus is still inactivated with chemicals which have the potential to damage epitopes on the antigens. While the use of the cell culture method avoids the use of embryonated eggs, there are new regulatory hurdles (clearance of adventitious agents) along with the limitations of traditionally produced egg vaccine due to the similarities in the process.

### Recombinant vaccines:

DNA vaccines encoding the HA and NP genes of influenza virus have been evaluated in mouse challenge models (Williams et al., 2002; Kemble and Greenberg, 2003). Vaccination with DNA encoding the NP gene resulted in protection from challenge with a heterologous influenza strain (Montgomery et al., 1993). Protection from homologous virus challenge was accomplished in mice after vaccination with DNA encoding HA. Antibody responses induced by vaccination with DNA resulted in long lived titres in mice (Ulmer etal., 1993). U.S. patent 4,357,421 disclose the production of a synthetic influenza hemagglutinin gene which is a double-stranded complementary DNA (cDNA) copy of a given specific influenza vRNA and which is capable of insertion into a bacterial plasmid engineered to ensure translation through the inserted DNA and expression of a polypeptide which will act as an influenza vaccine.

DNA vaccines are obviously not dependant on eggs or mammalian cell culture. However, most studies have presented encouraging results only in mice (Montgomery etal., 1993; Ulmer etal., 1993; and Williams etal., 2002). Reports of promising results in larger animals are very hard to find. A M2-NP DNA that worked well in mice exacerbated disease following challenge in a pig model (Heinen etal. 2002). While the potential exists for a DNA vaccine for influenza, there are still the safety issues that will continue to be a problem with this approach to vaccination. Some examples of DNA based vaccines are as follows:
- VGX has developed VGX-3400, a DNA based universal flu vaccine targeting variable influenza HA gene: [H1, H2, H3, and H5 (Avian)] along with conserved regions of NA and M2e-NP genes. All constructs were made using SynCon™ technology. Preclinical evaluation has been initiated at the University of Pennsylvania (USA) and an IND application was filed in May 2008.
- Patent W02005116270, assigned to Vical discloses a DNA vaccine containing the hemagglutinin gene of the H5N1 avian influenza virus A/Vietnam/1203/04 and the genes which are not so subject to mutation -- the nucleoprotein, NP and matrix protein, or M2e. It is formulated using Vicals's VAXFECTIN technology. In August 2007, Vical initiated a phase I trial of the vaccine in healthy volunteers in the USA. This patent does not suggest use of the PB2 gene in the gene construct.
- Novartis is developing a gene based vaccine for the prophylaxis of influenza using its proprietary drug delivery technology (This technology was formerly being developed by PowderJect. It has a gas driven apparatus for accelerating particles coated with a genetic material into a target, patent US5865796). The vaccine will utilize a multi vector strategy encoding both variable and conserved gene antigens. This is currently in preclinical stage.

- Patent WO2005035771, assigned to Pfizer covers nucleic acid constructs (vectors) that provide enhanced expression of heterologous coding sequences in mammalian host cells (formerly being developed by PowderMed). Pfizer is developing a DNA based vaccine, PF 4522625, for the prevention of seasonal influenza virus infection. The vaccine comprises the HA gene from the A/Panama/2007/99 influenza strain, cloned into PowderMed's (now Pfizer's) vaccine vector, pPJV1671, and is designed to be administered using PowderMed's (now Pfizer) proprietary needle free Particle mediated Epidermal Delivery system. Phase I evaluation is underway. The limitation is that the vaccine contains only the HA gene.
- Patent US7479285, assigned to Dynavax Tech Corp, discloses a universal influenza vaccine. It pertains to methods of modulating an immune response to one antigen by administering another antigen in conjunction with an immunostimulatory polynucleotide. The vaccine links immunostimulatory DNA sequences (ISS) to the key influenza antigen nucleoprotein (NP), an antigen that varies little between viral strains from year to year. NP-ISS are linked to the extracellular domain of matrix protein 2, M2e. Preclinical studies are ongoing in the USA.
- WO2004004758, assigned to Lipoxen discloses an influenza vaccine generated using the company's proprietary liposomal technology, ImuXen, which incorporates plasmid DNA in conjunction with its related protein into liposomes. The influenza vaccine liposome contains DNA that expresses influenza protein, plus the protein form of the antigen, co-formulated in the same particle. The formulation enables the delivery of multiple vaccine strains where each liposome acts as a monovalent vaccine. Preclinical evaluation is underway in UK.

Recombinant subunit protein vaccines have been proposed as the solution for many conventional vaccines. Recombinant protein production allows strict quality control of all vaccine components and more straightforward quantitation of lot-to-lot variation. This technology base has also been investigated for influenza vaccines. Expression systems based on *E.coli,* yeast, insect cells and mammalian cells have been utilized. The development of recombinant subunit protein vaccines for influenza is an attractive option because the need to grow virus is eliminated. Numerous studies have been reported regarding the testing of recombinant subunit protein vaccine candidates in animal models and only a few have been tested in human clinical trials. Two major problems have hampered the development of influenza vaccines based on recombinant proteins. Many a times it is difficult to express proteins in their native form and the expression levels are also low. For example, HA, the primary component of influenza vaccines has proven to be a difficult protein to express as a recombinant. Expression in Pichia of a membrane anchorless HA molecule has been reported (Saelens et al., 1999). While the expressed HA protein had appropriate structure based on antibody binding and resulted in partial protection of mice, the product was not completely uniform in nature. The N-terminus was variable due to variable processing and the glycosylation pattern was also heterogenous. Despite statements that the Pichia expressed HA protein has potential as a vaccine candidate there is no indication that this effort has been carried on for testing in humans.

The baculovirus expression system has also been investigated as a system for the production of recombinant influenza protein vaccine. An early report on the expression of full length HA using baculovirus expression system resulted in HA being localized on the surface of the insect cells (Kuroda etal., 1986) Further studies were reported on the expression of soluble HA from baculovirus expression system (Valandschoot etal., 1996)) This report on soluble baculovirus expressed HA, determined that although the protein had some native like characteristics but it was mostly aggregated. Therefore it failed to provide any protection in a mouse model. The recombinant baculovirus expressed HA protein under development by Protein Sciences Corporation (PSC Meriden, US patent US6224882) represents the most advanced recombinant influenza protein vaccines to date (Flubloc). The HA expressed by PSC represents the full length molecule and results in the localization of HA protein on the host insect cells. The HA is purified through a series of steps following extraction from the membrane. An H5-HA vaccine based on this methodology has been evaluated in human clinical trials (Treanor etal. 2001). The recombinant H5 vaccine was modestly immunogenic at high dose. The results of this study suggested that baculovirus-expressed H5 HA can induce functional antibodies in individuals who have not had prior exposure to H5 viruses, but that further studies to improve the immunogenicity of the vaccine are needed.

Virus based delivery of flu antigens has also been investigated. PCT application WO2006063101 assigned to the University of Pittsburg provides for the E1/E3 deleted adenovirus serotype-5 based vectors that express codon-optimized HA gene from A/Vietnam/1203/2004 influenza virus. This is currently in preclinical stage. However, this construct only contains the HA gene.

Another viral expression vector widely being evaluated is vaccinia virus. WO2008061939 (Paul Ehrlich), discloses use of MVA, for preparing influenza vaccines, most particularly from H5N1 strain. The invention pertains to influenza vaccine that preferably comprises of HA, NA, M1, M2 and NP genes of A/Vietnam/1194/04 strain of H5N1 avian influenza virus. There are claims directed to A/Vietnam/1203/04. In one of the preferred embodiments the heterologous gene sequence in MVA at DelIII can be in any combination of HA, NA, NP, M1, M2. The P11 promoter is one of the preferred promoters used in the invention. This application however does not disclose use of Influenza Polymerase gene in the construct and also there is a lack of specific disclosure of all the genes being cloned in the DelIII site of MVA.

Other alternative approaches for influenza vaccines include:
- Virus-like particles (VLP) produced in baculovirus expression system has been reported (Latham and Galzara, 2001). This methodology is currently being pursued by Novavax. Patent US20070184526, assigned to Novavax, covers VLPs comprising influenza M1, HA and NA proteins and their formulations. Novavax has developed a VLP based vaccine against H5N1 A/Indonesia/05/2005 virus and completed Phase I//IIa human clinical trials which show favorable results.
- Patent WO2006/069262, assigned to Vaxinnate claims a fusion protein between M2e from A/Vietnam/1203/04 and Salmonella typhimurium flagellin (fljB) which is a ligand for TLR5 (Toll-like receptor 5). Preclinical studies are on going in USA. However, the use of TLR agonists as vaccine adjuvants has been disappointing, at least with respect to the generation of T cell responses.
- Patent US7179645, assigned to Antigen Express, discloses expressing specific influenza HA (H5N1) antigens along with antigen presentation enhancing hybrid polypeptides. The vaccine could be used to prime T-helper cell responses to a recombinant H5 hemagglutinin protein or may be used as a stand alone vaccine. This is currently in preclinical stage. The limitation is that the vaccine contains only the HA gene.
- Acambis is developing a universal Flu vaccine named ACAM-FLU-A. It is a recombinant vaccine that uses a hepatitis B core protein to deliver M2e, the extracellular domain of the ion channel protein M2e. The vaccine contains Antigenics Inc., QS 21 adjuvant. A US phase I trial of the vaccine in healthy subjects was initiated in July 2007.
- WO2008085557 assigned to Alphavax Inc., discloses alphavirus replicon particle (ARP) preparations for enhancing the immune systems's response to a concurrently administered HA antigen. AlphaVax is developing influenza vaccines using its alphavaccine (alpha virus vector) technology. The vaccine in the program contains hemagglutinin gene from a single strain of influenza. The company has initiated the first of several clinical trials evaluating alphavaccines for influenza. The first alphavaccine for influenza contains the hemagglutinin gene from the A/Wyoming H3N2 strain of influenza. Subsequent trials will test additional vaccine candidates for potentially pandemic strains of influenza.
- VLP Biotech is developing a universal influenza vaccine. The vaccine is based on the influenza M2 protein in combination with the proprietary VLP vaccine platform. Preclinical studies are underway in USA.
- The National institute of Allergy and Infectious Disease (NIAID) is developing, in preclinical investigations, a vaccine to prevent infection by influenza A. The vaccine is a live attenuated, reassortant influenza A virus bearing a mutant polymerase basic 2 (PB2) gene.
- Vaxin Inc. is developing an intranasal H5N1 flu vaccine which delivers HA gene to the nasal mucosa via a non-replicating adenovirus vector. Vaxin uses the PER.C6© cell line, licensed from the Dutch biotechnology company Crucell©, as the manufacturing substrate for production of adenovirus-vectored vaccines. Phase I clinical trials for this H5N1 influenza vaccine have been completed. (www.curevents.corn/vb/showpost.php?p=616437&postcount=107). The limitations of such adenovirus based vaccine would be to keep on changing cloned HA gene every year depending upon the circulating strain at that time. In addition, a) adenovirus has a limited capacity to take up heterologus DNA and thus unable to accommodate several antigens and b) pre-existing immunity in 60-80% of the population, to adenovirus would limit the efficacy of adenovectorised vaccines.

Inspite of all these efforts, it is however unclear whether it will be feasible to completely replace conventional influenza vaccine based entirely on surface proteins (HA and NA), with the influenza vaccines based on internal viral proteins such as nucleoprotein or matrix protein. This is mainly because neutralizing antibodies to viral surface proteins play a crucial role in preventing establishment of infection whereas cytotoxic T lymphocytes to viral internal proteins will clear cells that are already infected. Thus it will be appropriate to include both viral surface and internal proteins in a vaccine construct. Although there are prior reports on use of highly conserved PB2 antigen in a vaccine construct, none of the prior arts suggest the antigen combination and process of the present invention.

In order to meet the challenges of next generation influenza vaccines, we have developed a new influenza vaccine that will combine both the surface and conserved internal viral proteins in a viral vector that can be produced in an easily available cell culture system. Some of the prominent advantages of the vaccine of the current invention over prior art are:
1. The incorporation of antigens on a yearly basis from the circulating strain of virus need not be required as the invention makes a "universal vaccine" for seasonal and pandemic flu which would be able to protect against different influenza strains. For this purpose, two internal genes of avian flu virus, M2 ectodomain gene and PB2 gene have been incorporated in MVA. The extracellular part of the M2 protein is remarkably conserved. No amino acid change has been noticed in the extracellular domain of M2 protein since the first human influenza A strain isolated in 1933.
2. The MVA based vaccine will overcome all the shortcomings of an egg based vaccine as the recombinant MVA viruses are grown in BHK21 cells rather than eggs.
3. As in the invention, a replication deficient vector is used; the problems of formalin inactivation and denaturation of antigenic epitopes have been avoided
4. The delivery of the MVA based vaccine will be done both by intranasal and intramuscular routes, priming by intranasal route and booster by intramuscular route or vice versa so as to generate both systemic and mucosal immune response.

### SUMMARY OF INVENTION

The present invention provides composition and method to prepare a universal influenza vaccine. The present invention is directed towards a universal influenza vaccine based on recombinant modified vaccinia Ankara (MVA) virus.

In accordance with these and other objects, the present invention relates to a recombinant modified vaccinia Ankara (MVA) virus comprising and capable of simultaneously expressing a cassette of at least four foreign genes from influenza virus, specifically an avian influenza virus, wherein the said genes are inserted at a non-essential site preferably DelIII, within the MVA genome, wherein each of the said foreign genes are under the transcriptional control of a individual single copy or multiple copies of the same promoter or multiple promoters.

In another embodiment the current invention relates to a recombinant modified vaccinia Ankara (MVA) virus comprising and capable of simultaneously expressing a cassette of not less than two foreign genes from influenza virus, specifically an avian influenza virus, wherein the said genes are inserted at a non-essential site preferably DelIII, within the MVA genome, wherein the said foreign genes are under the transcriptional control of a individual copies of a single promoter, with the provision that at least one foreign gene is either PB2 or M2e.

The invention further concerns influenza gene(s) expression cassette comprising promoters; vectors comprising said expression cassette as well as pharmaceutical compositions.

### BRIEF DESCRIPTION OF THE DRAWINGS

- **Fig.1** :: 1% agarose gel showing the presence of commercially synthesized HA, NA and PB2 genes in plasmids P8, 4, 7 (Double digestion).
- **Fig 2** :: 1% agarose gel showing the presence of commercially synthesized M2e and EGFP genes in plasmids P5 and 6 (Double digestion).
- **Fig 3** :: 1 % agarose gel showing a band of MVA genomic DNA.
- **Fig 4** :: Diagramatic representation of the construction of Transfer plasmid, p2.BRC/V/A9.
- **Fig 5** :: 1 % agarose gel showing the presence of cloned HA, NA, M2e, PB2, EGFP, right flank, left flank genes in the transfer plasmid and excision of the flu gene cassette by digestion with SnaBI.
- **Fig 6** :: Confirmation by PCR for the presence of recombinant MVA (having HA, NA, M2e, PB2 genes of H5N1) in the mixed progeny virus obtained after transfection. (Lane 1: 1Kb ladder; Lane 2, 4, 6, 8 : PCR of HA, NA, M2e, PB2 respectively from wild type MVA; Lane 3, 5, 7, 9: PCR of HA, NA, M2e, PB2 respectively from recombinant MVA)
- **Fig 7** :: The figure shows titre of anti influenza virus IgG in serum sample on day 28, 43, 71, 85 following intramuscular inoculation of Rec MVA in mice at 1.0 x 10⁸ pfu/mouse.
- **Fig 8** :: The figure shows titre of anti influenza virus IgG in serum sample on day 43, 71, 86 following intranasal inoculation of Rec MVA in mice at 1.0 x 10⁸ pfu/mouse.
- **Fig.9** :: The figure shows anti influenza virus IgA in bronchoalveolar lavage following inoculation of Rec MVA in mice at 1.0 x 10⁸ pfu/mouse.
- **Fig 10** :: Lymphoproliferation assay for cell mediated immunity following inoculation of Rec MVA in mice at 1.0 x 10⁸ pfu/mouse.

### DESCRIPTION OF INVENTION

The invention concerns influenza vaccine derived from incorporation of a combination of influenza genes in the genome of modified vaccinia Ankara (MVA) virus. The constructs of the present invention are not yet known in the art.

The present invention is not limited to the particular process steps and materials disclosed herein, but are extended to equivalents thereof as would be recognized by those skilled in the relevant arts. It should be understood that the terminology employed herein is used for the purpose of describing particular embodiments only and is not intended to be limiting.

### Definitions

### Modified vaccinia Ankara (MVA) virus

Modified vaccinia Ankara (MVA) virus is a 177kb dsDNA orthopoxvirus. It is a highly attenuated strain of vaccinia virus which was developed by > 570 serial passages in chicken embryo fibroblasts (CEFs ), during which it suffered six major deletions of DNA (namely deletion I, II, III, IV, V, and VI) totaling 31000 base pairs (Antoine et al., 1998). Deletion III (DelIII) site is one of the most commonly site in the MVA genome, used for the insertion and expression of the foreign sequences The complete genomic sequence of the modified vaccinia Ankara (MVA) virus and a comparison with other orthopoxviruses can be found in Virology 244:365-396. As a result of the serial passages, MVA virus has become severely host cell restricted to avian cells and replicates poorly in human and most other mammalian cells. Because of extreme attenuation, no adverse effects were reported even when high doses of MVA were given to immunodeficient non-human primates (Stittelaar *et al.,* 2001). There is exhaustive clinical experience using MVA for primary vaccination of over 120,000 humans against smallpox. During extensive field studies, including high risk patients, no side effects were associated with the use of MVA vaccine (Mayr et al., 1987; Stickl et al., 1974). Although MVA is highly attenuated, but it still maintains good immunogenicity. (Meyer *et al.,* 1991).

No MVA replication in human cells has been noticed since no assembly of mature infectious virions takes place following infection. Nevertheless, MVA is shown to express viral and recombinant genes at high levels even in non-permissive cells (mammalian cell lines) and thus has been considered as an efficient and exceptionally safe gene expression vector (Sutter and Moss, 1992). To further exploit the use of MVA, foreign genes have been introduced by DNA recombination into the MVA strain without altering the genome of the MVA virus.

### Blu-MVA

Blu-MVA is a recombinant MVA virus having β-gal gene inserted in the Del III site in its genome

### Baby hamster Kidney cell line (BHK21)

BHK21 cell line (Baby hamster Kidney cells), as used herein, is a fibroblast cell line derived from the kidney of hamster and established in 1961 by I. A. Macpherson and M. G. P. Stoker. The BHK21 cell line is susceptible to vaccinia virus, Vesicular stomatitis virus, Human adenovirus 25, reovirus 3 and Human poliovirus 2. This line has been used as a host for transfection with expression vectors containing desirable genes and marker DNAs.

### Enhanced Green Fluorescent Protein

EGFP, as used herein, refers to enhanced green fluorescent protein, a powerful reporter molecule used for monitoring gene expression, protein localization and protein-protein interaction. Several mutant variants of GFP are now available differing in absorption, emission spectra and quantum yield. Enhanced GFP (EGFP) is one such mutant of GFP containing a Phe-64-Leu and Ser-65-Thr mutation. It is being used extensively as it offers higher-intensity emission after blue light excitation with respect to wild type GFP. EGFP has emerged as an ideal molecule for fluorescence-activated cell sorting and other studies. (Canella etal. 2000)

### Promoters

A promoter is a region of DNA that facilitates the transcription of a particular gene. Promoters are typically located near the genes they regulate, on the same strand and upstream (towards the 5' region of the sense strand). For expression of heterologous genes in modified vaccinia Ankara (MVA) virus, it is essential to use poxvirus promoters because cellular and other viral promoters are not recognised by the MVA transcriptional apparatus. Strong late promoters like p11 or pCAE are preferable when high levels of expression are desired in MVA.

### Kozak sequence

Kozak sequence is the consensus sequence for initiation of translation in vertebrates. This sequence in an mRNA molecule is recognized by the ribosome as the translational start site, from which a protein is coded by that mRNA molecule

### Non essential regions of modified vaccinia Ankara (MVA) virus

Non essential regions according to the present invention may be selected from (i) naturally occurring deletion sites in the MVA genome with respect to the genome of the vaccinia virus or (ii) intergenic regions of the MVA genome. The term intergenic region refers preferably to those parts of the viral genome located between two adjacent genes that comprise neither coding nor regulatory sequences. However, the insertion sites for the incorporation of the heterologous nucleic acid according to the invention (non-essential region) are not restricted to these preferred insertion sites since it is within the scope of the present invention that the integration may be anywhere in the viral genome as long as it is possible to obtain recombinants that can be amplified and propagated in at least one cell culture system. Thus, a non-essential region may also be a non-essential gene or genes, the functions of which may be supplemented by the cell system used for propagation of MVA.

### Universal influenza vaccine

Universal influenza vaccine refers to an influenza vaccine intended to afford protection against the viruses causing seasonal as well as pandemic flu. Accordingly, two internal genes of avian flu virus, M2 ectodomain gene and PB2 gene have been incorporated in the non-essential regions, of the MVA genome. This recombinant virus is used for the preparation of the universal influenza vaccine. By virtue of this vaccine, the incorporation of antigens on a yearly basis from the current circulating strain of virus, to make a reassortant to be used in the preparation of the flu vaccine, need not be required.

### Present invention

In order to meet the challenges of next generation influenza vaccines, we have developed a new influenza vaccine that will combine both the surface and conserved internal viral proteins in a recombinant viral vector that can be produced in an easily available cell line.

### Cell line used in the present invention

MVA can be propagated in Chicken embryo fibroblast cells, BHK21 cells, African green monkey kidney cell lines CV-1 and MA104. Highest titre of MVA is obtained from Chicken embryo fibrablast cells but the preparation of CEF is cumbersome & requires particular cell culture experience. CV-1 & MA104 cells produced at best about one tenth the amount of virus compared to CEF cells. BHK21 cells are far more permissive for MVA propagation than CV-1 & MA104. BHK21 (C13) is the most preferred cell line as per the present invention.

### Vaccine

According to the invention the influenza gene may be selected from the group of H5N1, H5N3, H5N2, H5N7, H7N1, H7N3 and H9N2 sequences. Preferably the genes (s) are from H5N1. Amongst the preferred influenza genes are:
Hemagglutinin, HA is an antigenic glycoprotein found on the surface of the influenza viruses and is responsible for binding the virus to the cell that is being infected. There are at least 16 different HA antigens. These subtypes are labeled H1 through H16. The first three hemagglutinins, H1, H2 and H3 are found in human influenza viruses. This attachment is required for efficient transfer of flu virus genes into cells. The ability of various influenza strains to show species-selectivity is largely due to variation in the hemagglutinin genes. Genetic mutations in the hemagglutinin gene that cause single amino acid substitutions can significantly alter the ability of viral hemagglutinin proteins to bind to receptors on the surface of host cells. Avian influenza virus HA binds alpha 2-3 sialic acid receptors while human influenza virus HA binds alpha 2-6 sialic acid receptors. Swine influenza viruses have the ability to bind both types of sialic acid receptors. A mutation at amino acid position 223 of the haemagglutinin receptor protein increases the virus' ability to bind to human receptors, and decreases its affinity for poultry receptors, making strains with this mutation better adapted to infecting humans. Amino acid residues at positions 226, 227 and 228 of the receptor binding pocket of HA appear to determine binding affinity to cell surface receptors and to influence the selective binding of the virus to avian (sialic acid -2,3-NeuAcGal) or human (sialic acid -2,6-NeuAcGal) cell surface receptors. The human A/HK/212/03 and A/HK/213/03 isolates retain the signature associated with avian receptor binding, but they have a unique amino acid substitution (Ser227Ile) within the receptor binding pocket. Researchers have found that the mutations at two places in the HA gene, identified as 182 and 192, allow the virus to bind to both bird and
human receptors. A single E190D mutation in the HA of the H5N1 virus can potentially switch its binding preference from alpha 2-3 sialic acid (avian specific) to alpha 2-6 sialic acid (human specific). This is what happened with the 1918 Spanish flu virus, it did not have any mutations at the amino acids 226 and 228 and therefore had an avian specific receptor binding pocket, but just a single mutation at amino acid 190 changed its receptor specificity to alpha 2-6 sialic acid (human specific). HA provides neutralizing antibody response.

Neuraminidase, NA is an antigenic glycoprotein enzyme found on the surface of the influenza viruses. It helps in the release of progeny viruses from infected cells. NA is a target antigen that induces influenza virus specific immune responses, i.e. antibodies and T cell that may contribute to cross-protection against different virus variants or subtypes.

M codes for the matrix proteins M1 and M2 by using different reading frames from the same RNA segment. M1 is a protein that binds to the viral RNA. M2 functions as an ion channel protein permitting the flow of protons from endosome into the virion interior to facilitate removal of M1 protein from RNPs during virion uncoating in endosome. The extracellular part of the M2 protein (M2e) is remarkably conserved. No amino acid change has been found in the extracellular domain of M2 protein from the first human influenza A strain isolated in 1933. M2e provides protection by antibody dependent cell cyotoxicity (ADCC) mechanism.

Polymerases (PB1, PB2: Basic polymerase 1&2, PA: Acidic polymerase). PB1 is best characterized of the three P proteins; it contains five sequence blocks common to all RNA-dependent RNA polymerases and RNA-dependent DNA polymerases. PB1 codes for the PB1 protein and the PB1-F2 protein. The PB1 protein is a critical component of the viral polymerase. The PB1-F2 protein is encoded by an alternative open reading frame of the PB1 RNA segment and contributes to viral pathogenicity.PB2 has cap-binding and endonucleolytic activities which are necessary for viral mRNA synthesis. PA is indispensable for proper plus-strand copy RNA and vRNA synthesis, but no specific function in these processes has been assigned to it.

Nucleoprotein, NP codes for nucleoprotein whose primary function is to encapsidate the virus genome for the purposes of RNA transcription, replication and packaging.

NS codes for two nonstructural proteins - NS1 and NEP. NS1 (Non structural protein 1) suppresses the interferon response in the virus-infected cell leading to unimpaired virus production. NS1 protein circumvents host defenses to allow viral gene transcription to occur. HSN1 NS1 is characterized by a single amino acid change at position 92. By changing the amino acid from glutamic acid to aspartic acid, researchers were able to annul the effect of the HSN1 NS1. This single amino acid change in the NS1 gene greatly increased the pathogenicity of the H5N1 influenza virus. NEP, nuclear export protein, formerly referred to as the NS2 protein, mediates the export of vRNPs.

One embodiment of the invention relates to a recombinant MVA virus that can be used as a universal vaccine against Influenza virus. The invention is specifically directed towards production of a vaccine against H5N1 influenza virus. The universal vaccine of the invention further relates to MVA virus that comprises and is capable of expressing a novel combination of the H5N1 avian influenza antigens.

According to one of the embodiment of the present invention, nucleotide sequences that code for some of the antigens from the H5N1 virus have been introduced into the genome of MVA, which can be used as a vaccine. The genes used may be selected from but not restricted to HA, NA, PB2 and M2e genes from A/Vietnam/1203/04 H5N1 virus. The invention may not be restricted to H5N1 viruses.

The genes cloned from the influenza virus may be under the control of any suitable promoter. The genes may be under the control of single or multiple copies of same or different promoters. P11 is the most preferred promoter under the control of which the influenza genes may be expressed. In a preferred embodiment, all the genes are under the control of their individual P11 promoter.

According to another embodiment of the invention, the recombinant MVA virus may also have a marker gene cloned along with the influenza genes. In a preferred embodiment, the marker gene used may be enhanced green fluorescent protein gene (EGFP). The marker gene may be under the control of one of the P11 promoter controlling the regulation of influenza genes. In a preferred embodiment, the EGFP marker may be under the control of a separate P11 promoter.

All these genes, including the genes from the influenza virus as well as the marker gene, may be cloned in one transfer plasmid. The cloning may be carried out by methods known in the art. The transfer plasmid may further have sequences from the MVA virus that would aid in the homologous recombination of the transfer plasmid into the genome of the MVA virus at any naturally occurring deletion. One of the preferred embodiments of the invention relate to a transfer plasmid having influenza genes, the marker gene and the "left flank" and "right flank" sequences from the DelIII site of MVA virus that would aid the homologous recombination of the transfer plasmid at the DelIII site of MVA.

Thus, the genes from the influenza virus may be cloned in any non-essential site, like for example naturally occurring deletion in the viral genome of the MVA virus, to produce a recombinant MVA virus, which may be used as a vaccine. Preferably the genes from the influenza virus may be cloned in the DelIII site of the viral genome, to produce a recombinant MVA virus to be used as a vaccine.

According to a preferred embodiment of the invention, there is provided a novel plasmid deposited at ......... under the accession number........

One further preferred embodiment of the invention provides a novel plasmid comprising at least 2 genes from an influenza virus, specifically an avian influenza, virus, wherein at least one gene is PB2 and/ or M2e. According to a more preferred embodiment, the novel plasmid of the invention comprises Hemagglutinin (HA), Neuraminidase (NA), Polymerase PB2 and extracellular part of the Matrix (M) protein (M2e) from an influenza virus, specifically an avian influenza virus. One further preferable embodiment of the invention provides that these genes may be under the control of separate P11 promoters.

One further preferred embodiment of the invention relates to a recombinant MVA virus comprising and capable of simultaneously expressing a cassette of not less than two foreign genes from influenza virus, specifically an avian influenza virus, wherein the said genes are inserted at the site of DelIII within the MVA genome, and the foreign genes are under the transcriptional control of a single promoter, with the provision that at least one foreign gene is either PB2 or M2e.

Another preferred embodiment of the invention relates to a recombinant MVA virus comprising and capable of simultaneously expressing a cassette of at least four foreign genes from influenza virus, specifically an avian influenza, wherein the said genes are inserted at the site of DelIII within the MVA genome, and the said foreign genes are under the transcriptional control of a single, multiple or multiple copies of the same promoter. According to one preferred embodiment of the invention, the said influenza genes may be under the control of separate P11 promoters.

The preparation of the recombinant MVA virus may be done in various systems. BHK21 cells are the most preferred system, for the preparation of the recombinant MVA virus.

According to a preferred embodiment of the invention, there is provided a method of preparing recombinant modified vaccinia Ankara (MVA) virus, comprising the steps of:
a) culturing a mammalian cell line,
b) growing the cell line to confluency,
c) infecting the cells with Blu-MVA virus,
d) transfecting the cells with the nucleic acid comprising influenza virus, specifically an avian influenza virus under the control of P11 promoter,
e) passaging the progeny virus to increase the titre of the recombinant MVA and
f) isolation of the recombinant virus.

For preparation of the recombinant MVA virus, preferably, BHK21 cells may be infected with the MVA virus using known techniques. After the infection, the infected cells may be transfected with the transfer plasmid comprising the genes from the influenza virus. The transfection may be done either by calcium phosphate method or liposomes or any other method known in the art. Appropriate controls may be used during the experiment. After the cpe is visible, the transfected cells may be scraped, pelleted and freeze thawed thrice so as to release the virus which may then be used to infect a fresh batch of the BHK21 cells. The recombinant MVA may be passaged nine times before making a stock. This may be done to increase the titre of recombinant MVA. This stock virus may be used to confirm the integration of foreign genes in the mixed progeny by PCR, to confirm the expression of heterologous genes by Western blot and to isolate the recombinant MVA by FACS, based on EGFP fluorescence. The recombinant MVA may then be purified.

The invention further relates to a method for immunization of humans/animals/birds in need of a prophylactically effective amount of the recombinant MVA. Said method according to the invention may comprise a composition which may also optionally contain carriers, additives, antibiotics, preservatives, diluents, salts, buffers, stabilizers, solubilizers and other materials well known in the art. It is necessary that these are "pharmaceutically acceptable" which means that these are non toxic material that does not interfere with the effectiveness of the biological activity of the MVA according to the invention. The characteristics of the carrier will depend on the route of administration. The pharmaceutical composition may further contain other agents which either enhance the activity or use in treatment. Such additional factors and/or agents may be included in the pharmaceutical composition to be applied for the method for immunization according to the invention to produce a synergistic effect or to minimize side effects. Techniques for formulation and administration of MVA according to the invention may be found in "Remington's Pharmaceutical Sciences" (Muck Publishing Company, Easton, PA, latest edition).

Another embodiment of the invention relates to a method of treatment of an infection caused by an influenza virus, by administration to a subject, of the immunogenic composition or vaccine comprising a recombinant modified vaccinia Ankara (MVA) virus comprising genes from influenza virus, specifically an avian influenza.

The method for immunization according to the present invention will make use of a prophylactically effective amount of the recombinant MVA. Vaccines of the invention may be used to treat both children and adults. A prophylactically effective dose further refers to that amount of the compound/ ingredient sufficient to result in inhibition of establishment of infection or amelioration of symptoms.

The vaccine of the invention may be available in form of kits. A kit or composition may be packaged (e.g. in the same box) with a leaflet including details of the vaccine e.g. instructions for administration, details of the antigens within the vaccine, etc. The instructions may also contain warnings e.g. to keep a solution of adrenaline readily available in case of anaphylactic reaction following vaccination, etc.

According to an embodiment of the invention, the vaccine of the invention may be formulated to combine the modified vaccinia Ankara (MVA) virus comprising influenza genes with other antigens such as diphtheria, tetanus, pertussis, polio, *Haemophilus influenzae,* Hepatitis, meningitis, Pneumococci, Streptococci, anthrax, dengue, malaria, measles, mumps, rubella, BCG, Japanese encephalitis, Rotavirus, smallpox, yellow fever, typhoid, Singles, Varicella, and others. Another embodiment of the invention provides that the vaccine of the invention may be administered substantially at the same time, at the same or a different site, as that of the administration of the vaccines comprising other antigens as mentioned above.

The mode of administration, the dose and the number of administrations can be optimized by those skilled in the art in a known manner. The immunization will be prophylactic. The vaccine of the invention may be suitable for administration via parenteral as well as non-parenteral routes. In one embodiment, the administration of the immunogenic composition of the invention may be by mucosal route. In a preferred embodiment, delivery of the MVA based vaccine may be done both by intranasal and intramuscular routes, priming by intranasal route and booster by intramuscular route or vice versa. In a most preferred embodiment, the vaccine may be administered by intranasal route to immunize the patient against influenza. The intranasal administration of the vaccine composition can be formulated, for example, in liquid form as nose drops, spray, or suitable for inhalation, as powder or as cream or emulsion. The composition can contain a variety of additives such as stabilizers, buffers, or preservatives. For simple application, the vaccine composition is preferably supplied in a container appropriate for distribution of the antigen in the form of nose drops or an aerosol. Other routes of administration of the vaccine of the invention that may be used are oral, buccal, pulmonary, topical, parenteral (including subcutaneous, intramuscular and intravenous), transdermal, ocular (ophthalmic), transmucosal, implant or rectal administration.

The detailed example which follows is intended to contribute to a better understanding of the present invention. However, it is not intended to give the impression that the invention is confined to the subject matter of the example.

### EXAMPLE

### 1. Transformation with plasmids having commercially synthesized influenza genes (HA, NA, M2e, PB2 and EGFP)

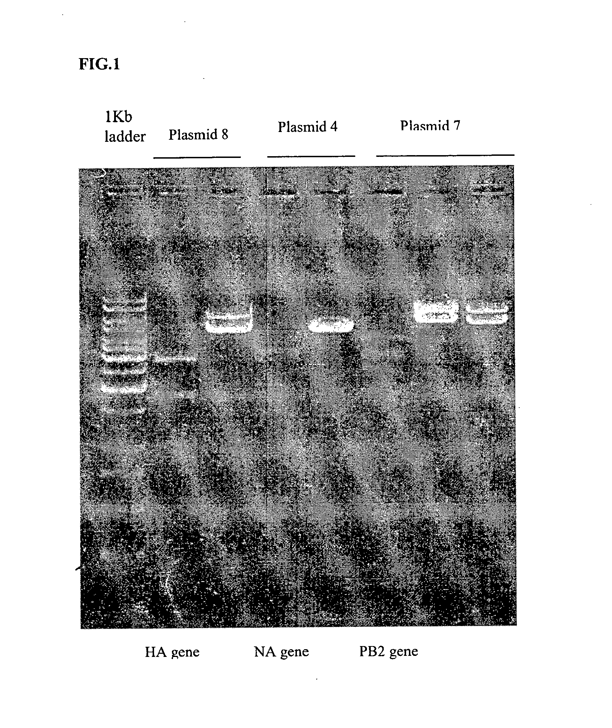

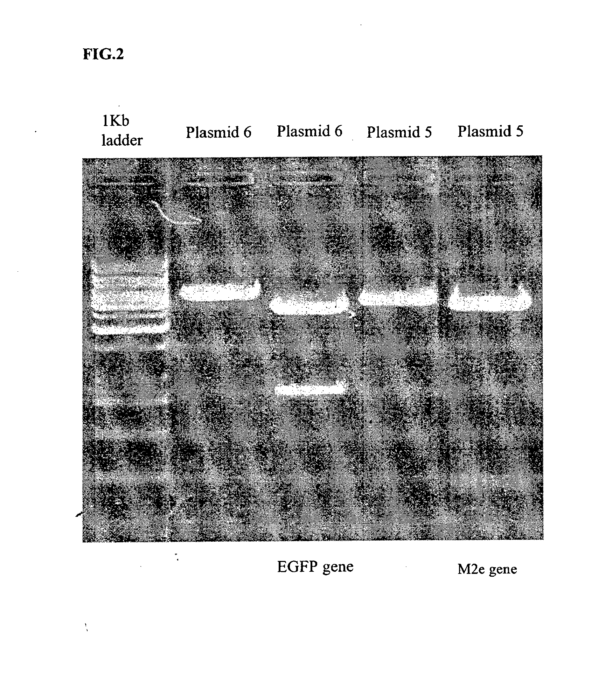

The Hemagglutinin (HA, Accession No.: AY818135), Neuraminidase (NA, Accession No.: AY818141), Matrix protein 2 ectodomain (M2e, Accession No.: AY651388) Polymerase basic subunit 2 (PB2, Accession No.: AY651719) genes of A/Vietnam/1203/04 strain of H5N1 and Enhanced Green Fluorescent Protein gene (EGFP, Accession No.: U57609) were procured after synthesis from a commercial source. Each of the genes had promoter p11 and ribosomal binding site kozak sequence in front of the gene. The artificially synthesized genes were cloned in pGA1/pUC-Kana/pPCR-Script/pGA4 to generate plasmids p8, p4, p5, p7 and p6 respectively. Each of these plasmids was used to transform *E.coli* DH5alpha competent cells (Maniatis, Sambrook etal, 2001). Two transformed colonies containing each of the plasmids were inoculated in a suitable nutrient medium and plasmids were isolated by using Qiagen kit as per the standard protocol provided by the manufacturer. The isolated plasmids were digested by appropriate restriction enzymes to confirm the presence of required genes (Figure 1 and Figure 2) and thereafter, stored at -20°C. The glycerol stocks of the plasmids were stored at -80°C in the form of a culture of *E. coli* DH5alpha transformed with each of the plasmids.

### 2. Culture of Baby Hamster Kidney (BHK21) cell line.

The BHK21 (C13) cell line was obtained from ATCC. The BHK21 cells were maintained by regular splitting and seeding in DMEM (Dulbeco's modified Eagle medium) media supplemented with 10% NBCS. Stock of BHK21 cells was also prepared side by side.

### 3. Growing the MVA virus

### 3.1 Revival of MVA and stock preparation of MVA.

MVA virus was revived in DMEM medium, by infecting BHK21 cells. However, any suitable medium may be used. It was passaged repeatedly in BHK21 cells till it reached a substantially high titre. The MVA infected BHK21 cells were harvested using standard methods and stored at -80°C.

### 3.2 Titration of MVA

As the MVA does not form plaque in BHK21 cells, its concentration was calculated by

Karber method to determine TCID₅₀/ml (Vaccinia Virus and Poxvirology: Methods and Protocols, By Stuart N. Isaacs, 2004).

### 4. Isolation of MVA genomic DNA.

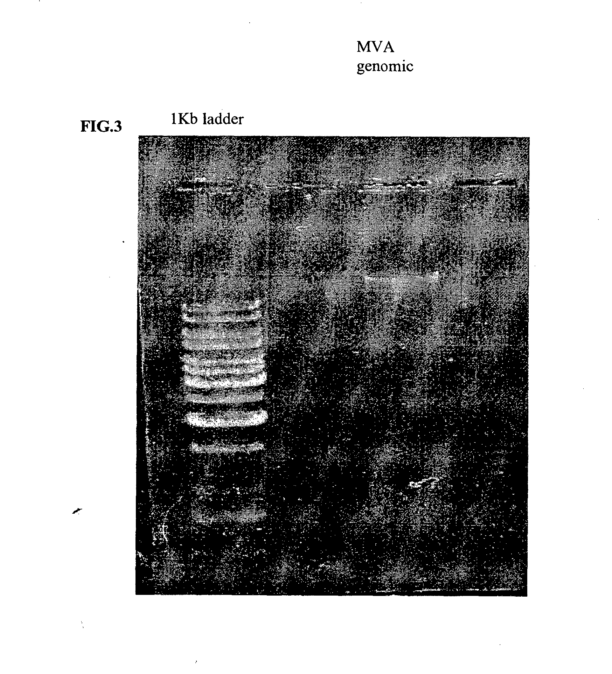

BHK21 (C13) cells were infected by MVA stock. After onset of CPE, infected BHK21 cells were harvested and used for MVA genomic DNA extraction (as per the protocol cited in Vaccinia Virus and Poxvirology: Methods and Protocols, By Stuart N. Isaacs). The pellet obtained was washed with 70% ethanol, air dried and dissolved in deionised sterilized water. The genomic DNA extracted was run on agarose gel (Figure 3).

### 5. Construction of transfer plasmid p2.BRC/V/A9.

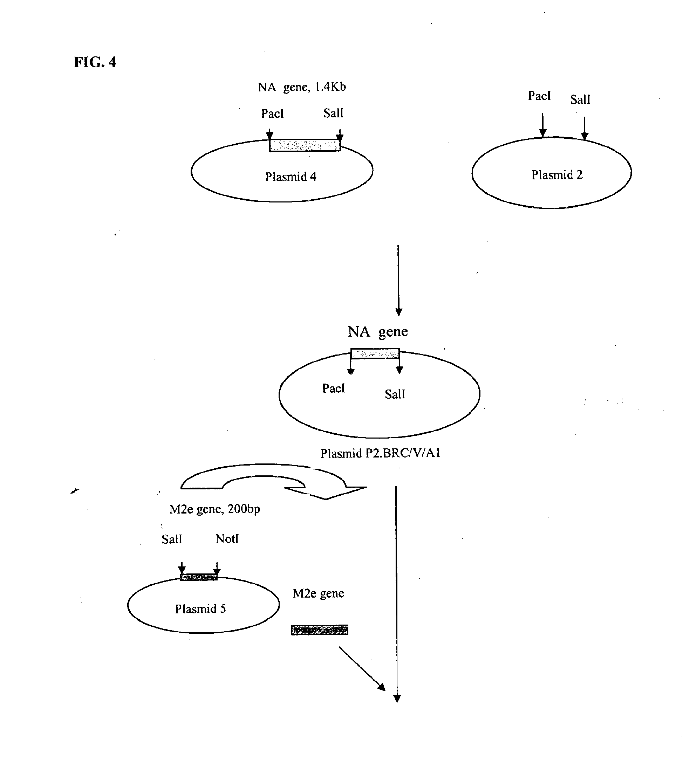

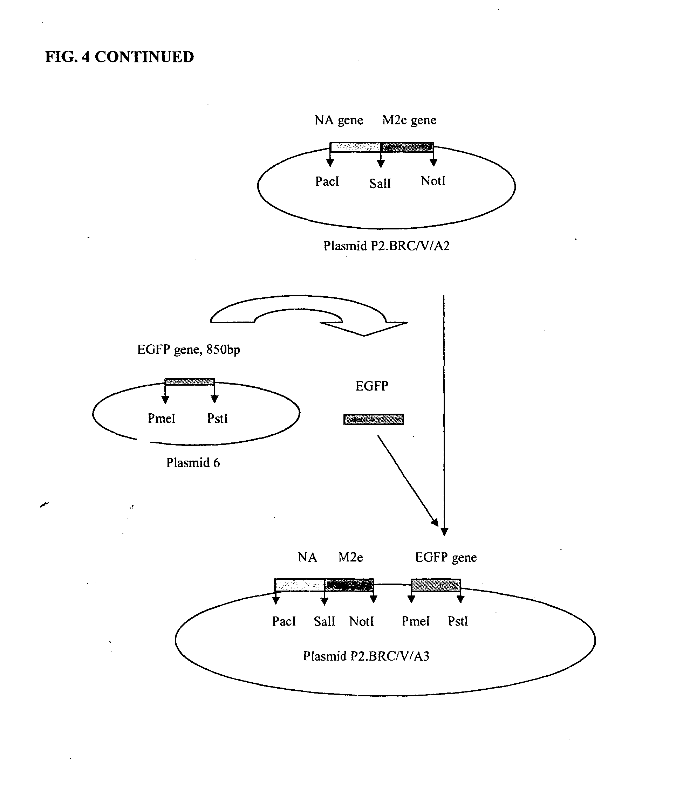

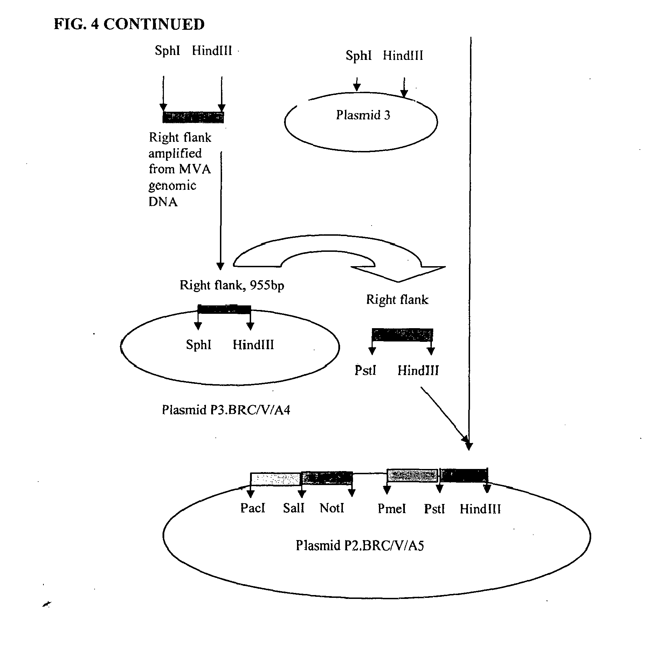

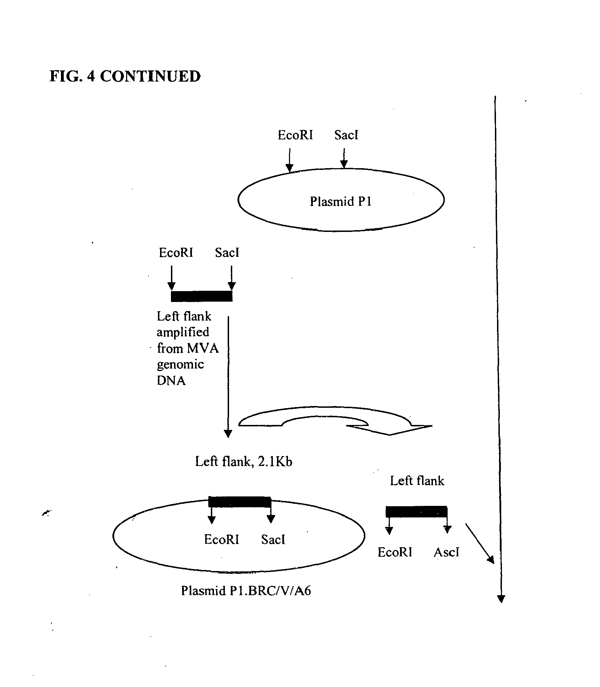

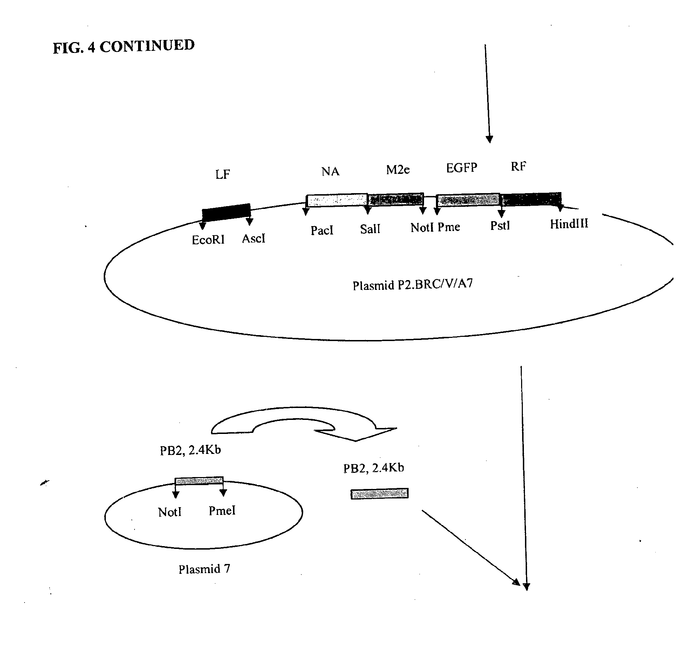

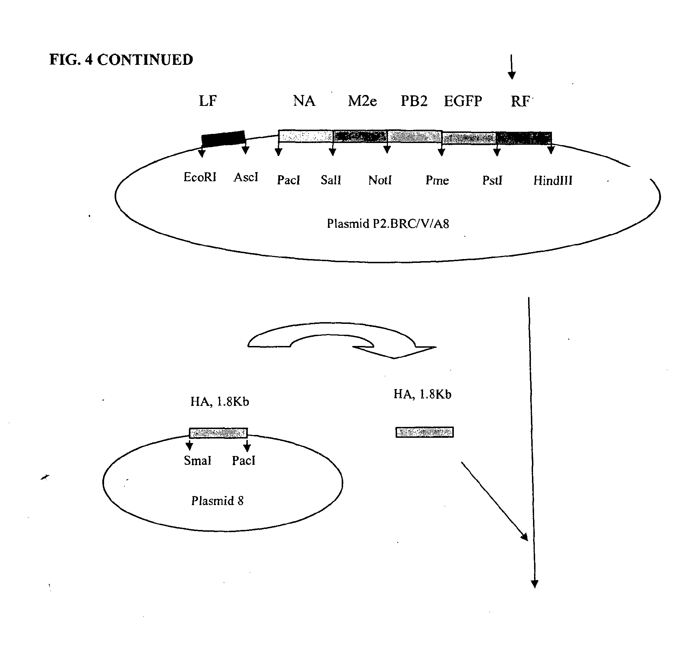

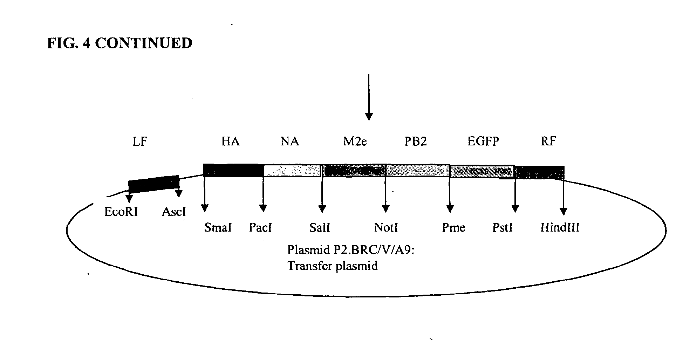

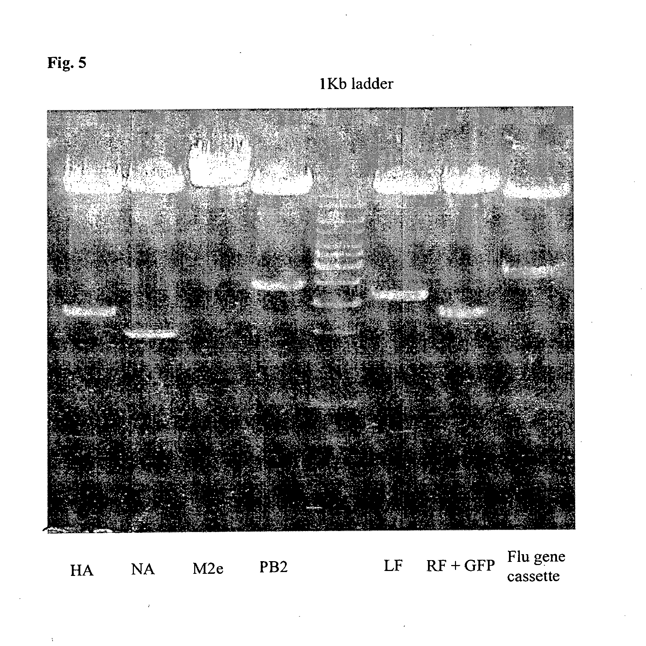

A transfer plasmid was constructed through a number of cloning steps (Figure 4). The final transfer plasmid has the HA, NA, M2e & PB2 influenza genes and the EGFP marker gene, each under the control of separate P11 promoter. The stretch of the genes in the transfer plasmid are flanked from both the sides by "left flank" and "right flank" sequences of the Del III site of MVA (Figure 5) that will aid in the homologous recombination of the transfer plasmid in the Del III site of the MVA viral genome.

### 6. Generation of recombinant MVA.

### 6.1. Excision of transfer cassette.

The transfer plasmid p2.BRC/V/A9 was digested by restriction enzyme SnaBI, (which has a site in both the left and right flanks) to excise the entire cassette containing the genes HA, NA, M2e, PB2, the marker gene EGFP along with the MVA flanks (Figure 5) The digested and gel purified cassette was eluted in sterile water.

### 6.2 Transfection with transfer cassette and full length transfer plasmid.

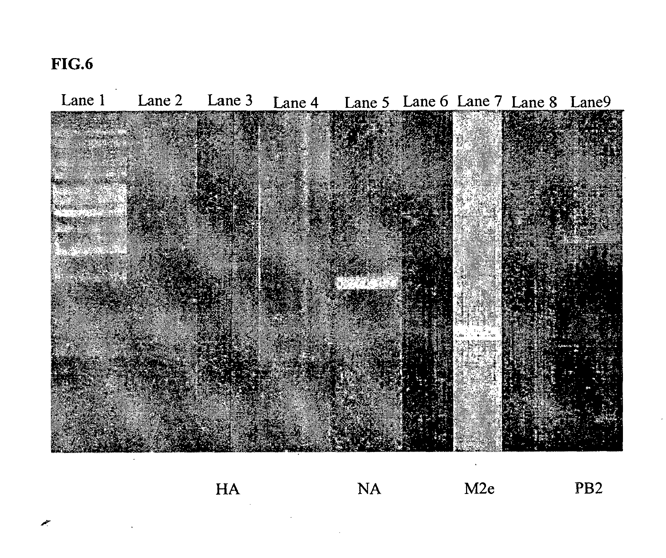

BHK21 cells were cultured in 60mm tissue culture dishes. At 80-90% confluency, the medium was removed, cells were washed with 1X PBS⁺⁺ (PBS containing 1% CaCl₂ and 1% MgCl₂) and infected with 1ml/ 60mm dish of 10⁻⁵ dilution of Blu-MVA (a recombinant MVA that has β-gal gene inserted in the Del III site) virus stock. During the 1 hr infection period, transfection mixes were made either by using the gene cassette (containing the H5N1 genes HA, NA, M2e, PB2, the marker gene EGFP and the MVA flanks) or by using the full length transfer plasmid. Transfection reagents used were either lipofectamine or Calcium Chloride. After 1hr incubation, virus was removed and the transfection was done by the methods known in the art. Following 3-4 days incubation at 37°C, the transfected cells were scraped, pelleted, subjected to three cycles of freeze-thaw to release the recombinant and wild type virus. This mixed population of the virus was used to infect fresh BHK21 cells. The progeny virus was passaged a few times in the BHK-21 cells before making a stock. This was done to increase the titre of recombinant MVA. This stock virus was used to confirm the integration of foreign genes in the mixed progeny by PCR (Figure 6) and to isolate the recombinant MVA by chromogenic detection.

### 7. Isolation of recombinant MVA

Chicken embryo fibroblasts (CEF) were prepared from 11 days old embryonated SPF eggs. The cells were infected with the stock of mixed recombinant and wild type progeny. The viral plaques were detected by an overlay of X-gal. White plaques indicating the recombinant virus were picked.

### 8. Amplification, purification and titration of recombinant MVA.

### 8.1 Amplification of recombinant MVA

Once the recombinant virus was isolated, it was amplified by using the BHK21 cells. The recombinant MVA was amplified by sequentially infecting T25 flask, T75 and T175 flask of BHK21 cells. Recombinant MVA infected cells grown in T175 were pelleted, dissolved in 10mM Tris, pH8 and stored at -80°C.

### 8.2 Purification of recombinant MVA

The cells infected with r-MVA were disrupted by sonication and centrifuged to remove cell debris. The supernatant was applied to a stepped sucrose gradient (concentration steps: 20%, 25%, 30%, 35% and 40%). The band of the purified virus was retrieved, pelleted and stored at -80°C.

### 8.3 Titration of recombinant MVA

As the MVA does not form plaque in BHK21 cells, its concentration was calculated by TCID₅₀/ml. BHK21 cells were seeded in a 96 well plate and infected with two fold serial dilutions of the recombinant MVA. The plate was incubated at 37°C in CO₂ incubator for 5 days. The TCID₅₀ value per ml was calculated as per Karber's method. (Vaccinia Virus and Poxvirology: Methods and Protocols, By Stuart N. Isaacs, 2004)

### 9. Immunogenicity studies of Recombinant MVA-Flu virus in BALB/c mice

One group of 6-8 weeks old BALB/c mice (n=6) were immunized with purified recombinant MVA-Flu virus at the dose rate of 1.0 x 10⁸ PFU/mouse through intramuscular route. Second group of 6-8 weeks old BALB/c mice (n=6) were immunized with purified recombinant MVA-Flu virus at the dose rate of 1.0 x 10⁸ PFU/mouse through intranasal route. Placebo groups were taken appropriately. Blood samples were collected before immunization from each mouse.

All animals in both the groups were given booster dose, by respective routes, of purified recombinant MVA-Flu virus (1.0 x 10⁸ PFU/mouse) at day 7, 28 and 50. Each mouse was bled on day 0, 28, 43, 71 and 85 post-initial immunization (86th day post-initial immunization in case of group immunized intranasally). BAL samples and spleen from each mouse were collected on the day of euthanasia.

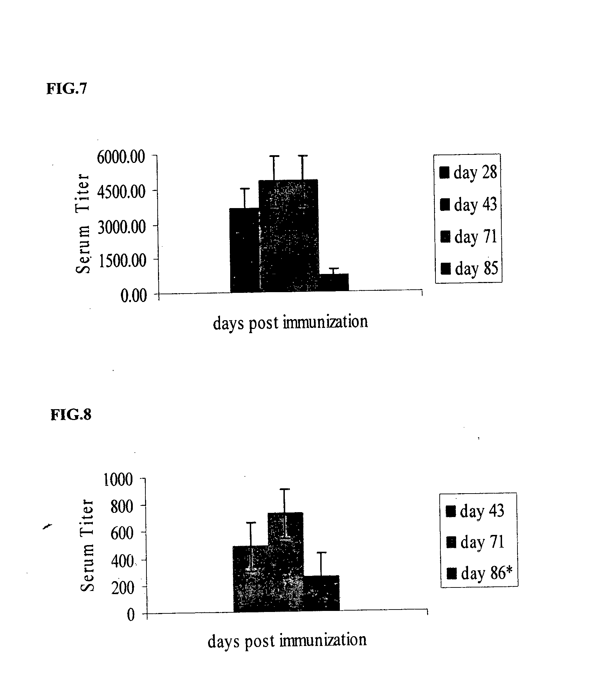

To evaluate influenza specific systemic humoral immune response, antibodies against influenza virus in serum samples of each mouse were determined by ELISA (Figure 7 and 8).

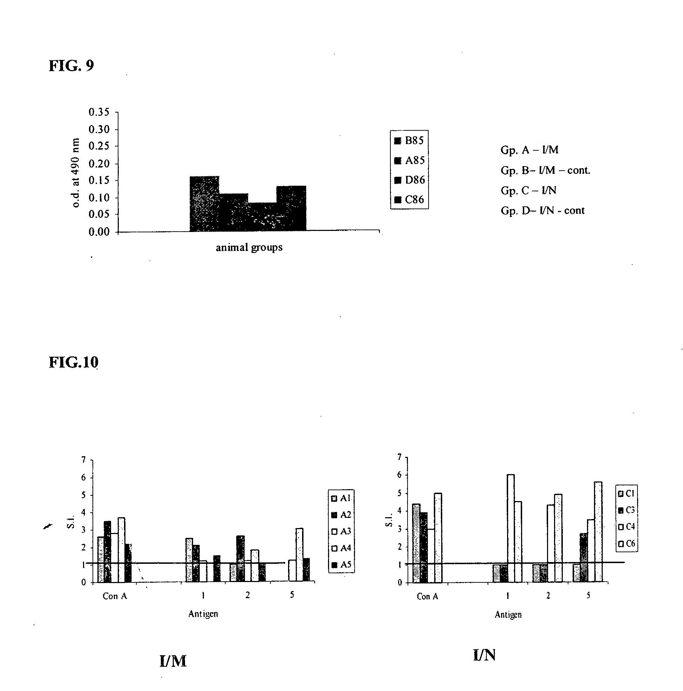

To evaluate influenza specific mucosal humoral immune response, antibodies against influenza virus in BAL samples of each mouse were determined by ELISA (Figure 9).

Lymphoproliferation assay (Current Protocols In Immunology, Vol I) using lymphocytes isolated from spleen was done to evaluate cellular immune response against influenza virus (Figure 10).

## Claims

1. A recombinant modified vaccinia Ankara (MVA) virus comprising and capable of simultaneously expressing the cassette of not less than 2 genes from influenza virus, wherein the said genes are inserted at the site of deletion III within the MVA genome, with the proviso that at least one gene is either PB2 and/ or M2e.

2. The recombinant modified Vaccinia Ankara (MVA) virus according to claim 1, comprising and capable of simultaneously expressing a cassette of at least 4 genes from an influenza virus, wherein the said genes are inserted at the site of deletion III within the MVA genome.

3. The recombinant modified Vaccinia Ankara (MVA) virus according to claim 1 or 2, wherein said influenza virus is an avian influenza virus, or wherein said influenza virus is an avian influenza virus selected from the group comprising of H5N1, H5N3, H5N2, H5N7, H7N1, H7N3 and H9N2, or wherein said influenza virus is H5N1, or wherein said avian influenza virus is A/Vietnam/1203/04 H5N1.

4. The recombinant modified Vaccinia Ankara (MVA) virus according to any one of the preceding claims, wherein the genes from the influenza virus are selected from the group comprising of Hemagglutinin (HA), Neuraminidase (NA), Matrix proteins (M1 and M2), Polymerases (PB1, PB2 and PA), Nucleoprotein (NP) and Non structural proteins (NS and NEP), or wherein the genes from the avian influenza virus are Hemagglutinin (HA), Neuraminidase (NA), Polymerase PB2 and extracellular part of the Matrix (M) protein (M2e).

5. The recombinant modified Vaccinia Ankara (MVA) virus according to any one of the preceding claims, wherein a marker gene is cloned along with the genes of the influenza virus, and/or wherein the marker gene is the Enhanced Green Fluorescent Protein (EGFP) gene.

6. The recombinant modified Vaccinia Ankara (MVA) virus according to any one of the preceding claims, wherein the genes from the influenza virus are under the control of single or multiple copies of same or different promoters.

7. The recombinant modified Vaccinia Ankara (MVA) virus according to any one of the preceding claims, wherein the influenza virus is an avian influenza virus and all the genes are under the transcriptional control of one promoter, or wherein the influenza virus is an avian influenza virus and each gene is under the transcriptional control of a separate promoter.

8. The recombinant modified Vaccinia Ankara (MVA) virus according to claim 6 or 7, wherein all the genes are under the transcriptional control of one promoter and said promoter is P11, or wherein each gene of the influenza virus is under the transcriptional control of separate and said separate promoters are P11 promoters

9. A method of preparing a recombinant modified vaccinia Ankara (MVA) virus according to any one of the preceding claims, comprising the steps of
a) culturing a mammalian cell line,
b) growing the cell line to confluency,
c) infecting the cells with Blu-MVA virus,
d) transfecting the cells with a nucleic acid comprising the influenza genes under the control of the P11 promoter,
e) passaging the progeny virus to increase the titre of the recombinant MVA and
f) isolating the recombinant modified vaccinia Ankara (MVA) virus.

10. The method as claimed in claim 9, wherein the mammalian cell line is BHK21.

11. A vaccine comprising the virus as claimed in any one of claims 1 to 8, optionally comprising other excipients, diluents and stabilizers.

12. The vaccine according to claim 11, which is in a form suitable for parenteral and non-parenteral administration.

13. The vaccine according to claim 11 or 12, which is in a form suitable for administration via intranasal, intramuscular and mucosal routes, or which is suitable for delivery via nasal route, in liquid form as nose drops or sprays, or via inhalation, as powder or as cream or emulsion.

14. The vaccine according to any one of claim 11 to 13, for use in a method of treatment of an infection caused by influenza virus.

15. A kit comprising the vaccine according to any one of claims 11 to 14 and a leaflet giving details of the vaccine, *e.g.*, instructions for administration and/or details of the antigens within the vaccine.
